(19) **Europäisches Patentamt / European Patent Office / Office européen des brevets**

(11) **EP 1 621 887 B1**

(12) **EUROPEAN PATENT SPECIFICATION**

(45) Date of publication and mention
of the grant of the patent:
**02.09.2009 Bulletin 2009/36**

(51) Int Cl.:
***G01N 33/558*** (2006.01)

(21) Application number: **05254766.8**

(22) Date of filing: **29.07.2005**

(54) **Analytical test strip with control zone**

Analytischer Teststreifen mit Kontrollzone

Bande de test analytique comprenant une zone de contrôle

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.07.2004 US 903108**

(43) Date of publication of application:
**01.02.2006 Bulletin 2006/05**

(73) Proprietor: **LifeScan, Inc.**
**Milpitas, CA 95035-6312 (US)**

(72) Inventors:
• **Matzinger, David Parkes**
**Menlo Park, CA 94025 (US)**
• **Guo, Sherry**
**San Jose, CA 95132 (US)**

• **Quraishi, Khalid Rashid**
**Sunnyvale, CA 94085 (US)**

(74) Representative: **Mercer, Christopher Paul et al**
**Carpmaels & Ransford**
**43-45 Bloomsbury Square**
**London WC1A 2RA (GB)**

(56) References cited:
**EP-A- 1 359 417       US-A- 5 712 170**
**US-A- 5 843 691**

• **RODRIC H. WHITE-STEVENS AND LON R. STOVER: "Interference by Ascorbic Acid in Test Systems Involving Peroxidase II Redox-Coupled Indicator Systems" CLINICAL CHEMISTRY, vol. 28, no. 4, 1982, pages 589-595, XP002351014**

## Description

## BACKGROUND OF THE INVENTION

1. Field of the Invention

The present invention relates, in general, to analytical devices and, in particular, to analytical test strips.

2. Description of the Related Art

A variety of conventional analytical tests strips for the determination of an analyte in a fluid sample are known. For example, analytical test strips for the determination (e.g., detection and/or concentration measurement) of glucose in a whole blood sample are widely employed by patients and their healthcare providers (see, for example, U.S. Patent No. 5,304,468).

Conventional analytical test strips are typically employed with an associated meter that detects an optical response (e.g., a colorimetric response) or an electrochemical response created on the analytical test strip by interaction between the analyte and a reagent composition present in or on the analytical test strip. Unfortunately, the proper functioning of such analytical test strips and their associated meters can be subject to a variety of deleterious interfering factors. For example, the analytical test strip's reagent composition can degrade over time leading to improper functioning of the analytical test strip. Similarly, portions of the meter can miss-function or the meter can be employing incorrect calibration codes. In addition, properties or constituents of the liquid sample itself can lead to a deleterious interference with the proper functioning of an analytical test strip and/or associated meter. Such deleterious interferences from the liquid sample itself are known as a "matrix effects."

In order to verify the proper functioning of a batch of test strips and associated meter, it is common for users to check one analytical test strip from the batch using a control solution that contains a predetermined amount of analyte. However, such checking is not only time consuming and cumbersome, but also wasteful, as the analytical test strip employed for the checking must be discarded. In addition, the control solution used for such a check may not reliably simulate or predict the matrix effects of the actual liquid sample that will be used with the analytical test strips.

Still needed in the art, therefore, is an analytical test strip for which the proper functioning can be verified in an expeditious and simple manner. In addition, such verification should take into consideration matrix effects of the fluid sample used with the analytical test strip.

## SUMMARY OF THE INVENTION

Embodiments of the present invention include analytical test strips whose proper functioning can be verified in an expeditious and simple manner. In addition, such verification takes into consideration fluid sample matrix effects.

An analytical test strip for the determination of an analyte (e.g., glucose) in a liquid sample (such as whole blood) according to an exemplary embodiment of the present invention includes a matrix, with the matrix having both a sample detection zone and a control zone(s). The sample detection zone includes a first reagent composition that reacts with analyte in the liquid sample to create a detectable sample response and is configured to receive a first portion of the liquid sample. The control zone(s) includes a second reagent composition and is configured to receive a second portion of the liquid sample. In addition, the second reagent composition creates a detectable predetermined control response when exposed to the second portion of the liquid sample. The predetermined control response, either alone or in combination with the sample response, can be employed to verify proper functioning of the analytical test strip and/or associated meter, or to provide a calibration factor for the analytical test strip.

Since analytical test strips according to embodiments of the present invention create both a sample response and a predetermined control response upon the application of a single fluid sample (for example, a patient's blood sample), time, effort and expense related to the use of separate control solutions is eliminated. In addition, since the sample detection zone and control zone(s) of analytical test strips according to the present invention are exposed to respective portions of the same fluid sample, effects of the fluid sample (i.e., "matrix" effects) are present in both the sample detection and control zones and, thus, can be accounted for in both the sample and predetermined control responses. Furthermore, since the sample detection zone and control zone(s) are integrated into a single analytical test strip, the use of an analytical test strip solely for verification purposes and the associated expense are avoided.

## BRIEF DESCRIPTION OF THE DRAWINGS

A better understanding of the features and advantages of the present invention will be obtained by reference to the following detailed description that sets forth illustrative embodiments in which the principles of the invention are utilized, and the accompanying drawings of which:

FIG. 1 is a simplified exploded perspective view of an analytical test strip according to an exemplary embodiment of the present invention;

FIG. 2 is a simplified perspective depiction of a web-based process for manufacturing analytical test strips according to various embodiments of the present invention;

FIG. 3 is an idealized graph of analyte concentration in a fluid sample on the x-axis versus response (either sample response or predetermined control response) on the y-axis;

FIG. 4 is a simplified exploded perspective view of an analytical test strip according to another exemplary embodiment of the present invention;

FIGs. 5A and 5B are K/S versus scan distance for analytical strips of Example 1 and K/S versus glucose concentration for sample detection zones, control zones and the difference therebetween, respectively; and

FIGs. 6A and 6B are K/S versus scan distance for analytical strips of Example 2 and K/S versus glucose concentration for sample detection zones and control zones, respectively.

## DETAILED DESCRIPTION OF PREFERRED EMBODIMENTS

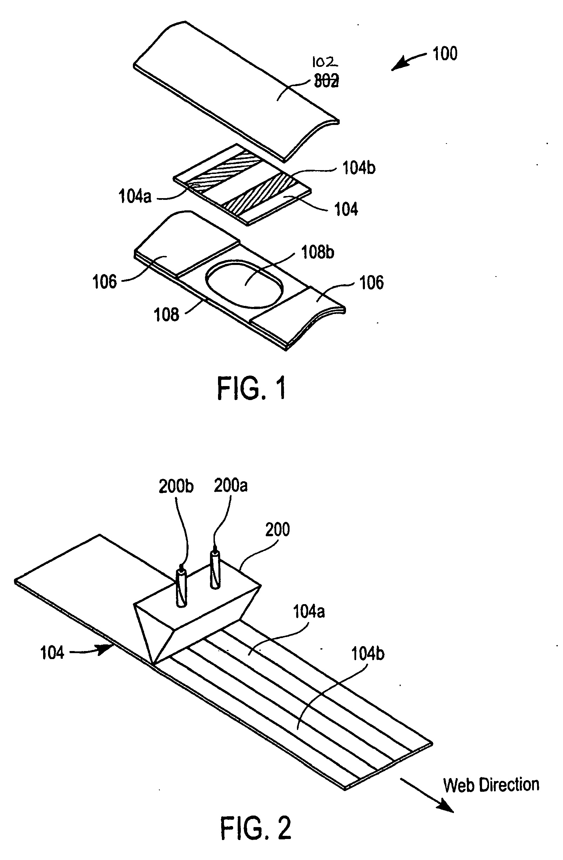

FIG. 1 is a simplified exploded perspective view of an analytical test strip 100 for the determination of an analyte (i.e., glucose) in a liquid sample (i.e., whole blood) according to an exemplary embodiment of the present invention. Although analytical test strip 100 is adapted for the determination of glucose in whole blood, once apprised of the present disclosure one skilled in the art will recognize that embodiments of analytical test strips according to the present invention can be adapted to the determination of other analytes (such as calcium, ketones, medications, etc.) and/or for analytes in other liquid samples (for example, a urine sample, a serum sample, a plasma sample, an interstitial fluid sample, etc.).

Analytical test strip 100 includes a liquid sample spreading layer 102, a matrix (e.g., a membrane layer) 104, pressure sensitive adhesive layer 106 and base layer 108. In the depiction of FIG. 1, pressure sensitive adhesive layer 106 is shown adhered to base layer 108. Liquid sample spreading layer 102 can be any suitable liquid sample spreading layer known to those skilled in the art including, but not limited to, liquid sample spreading layers formed from Porex material. Suitable liquid sample spreading layers are described in, for example, U.S. Patent Nos. 6,162,397 and 6,168,957. Liquid sample spreading layer 102 serves to transfer portions of a fluid sample applied thereto evenly across matrix 104.

Matrix 104 can be formed of any suitable material including, but not limited to plastics, membranes, fibrous mats, woven fabrics, gelatin, hydrogels and combinations thereof. Some examples of suitable matrixes (also referred to as "pads" or "testing pads") are described in U.S. Patent Nos. 4,900,666; 5,304,468; 5,902,731; and 5,968,836.

Pressure sensitive adhesive layer 106 can be any suitable pressure sensitive adhesive layer known to one skilled in the art including. Base layer 108 includes an aperture 108b through which matrix 104 is exposed and through which sample and predetermined control responses created on matrix 104 can be detected.

Matrix 104 includes a sample detection zone 104a with a first reagent composition that reacts with analyte in the liquid sample to create a detectable sample response (for example, a colorimetric response). The detectable sample response is dependent on the concentration of analyte in the liquid sample. Sample detection zone 104a is configured to receive a first portion of a liquid sample that has been applied on liquid sample spreading layer 102.

The first reagent composition included in sample detection zone 104a can be any suitable first reagent composition known to one skilled in the art. For the circumstance that the analyte of interest is glucose and the liquid sample is a whole blood sample, suitable reagents include, but are not limited to, a tetrazolium dye, an electron transfer agent (such as, for example, phenazine methosulfate) and an enzyme. Suitable reagents are also detailed in Examples 1 and 2 below and, for example, in U.S. Patent Nos. 4,935,346, 5,304,468, 6,162,397, and 6,168,957. Furthermore, once apprised of the present disclosure, one skilled in the art will recognize that components of the first reagent composition employed in the sample detection zone of analytical test strips according to the present invention will depend on the nature of the analyte and liquid sample being tested, as well as the means that will be employed to detect the sample response.

Matrix 104 also includes a control zone 104b with a second reagent composition that creates a detectable predetermined control response (for example, a predetermined colorimetric response) when exposed to a second portion of the liquid sample. Control zone 104b is configured to receive a second portion of the liquid sample that has been applied on liquid sample spreading layer 102.

The sample responses and predetermined control response are occasionally referred to as "detectable" responses since it is envisioned that these responses will be detected by a meter or other device that is associated with the analytical test strip. For colorimetric sample and predetermined control responses, such a meter would include a light source (such as Light Emitting Diode [LED]), a light detector, and suitable circuitry to enable the detection and analysis of the sample and predetermined control responses. Once apprised of the present disclosure, one skilled in the art could readily modify conventional meters to perform such functions.

The second reagent composition included in control zone 104b can, for example, employ the same general chemistry (for example, the same dye(s), enzymes, buffers, etc.) as the first reagent composition of sample detection zone 104a. However, the second reagent composition will also typically include supplemental reagent components and/or modified ratios of reagent components such that a predetermined control response is created when the second reagent composition is exposed to the second portion of the liquid sample.

Sample detection zone 104a and control zone 104b can be formed on matrix 104 by any suitable technique known. For example, FIG. 2 depicts a web-based technique for applying reagents to a matrix 104. In the web-based technique depicted in FIG. 2, matrix 104 is a membrane that has been previously impregnated with reagent components that are common to both sample detection zone 104a and control zone 104b (e.g., enzymes and buffer reagent components). As previously impregnated matrix 104 moves in the "web direction" as indicated in FIG. 2, additional reagent components are applied to matrix 104 using slot coater head 200 and nozzles 200a and 200b. For example, a dye solution can be applied through nozzle 200a to form sample detection zone 104a, while a dye and glucose solution can be applied through nozzle 200b to form control zone 104b. In this circumstance, the dye solution together with the previously impregnated enzymes and buffer reagents are employed to form the first reagent composition, while the dye and glucose solution together with the previously impregnated enzymes and buffer reagents are employed to form the second reagent composition.

Referring again to FIG. 1, the predetermined control response of control zone 104b can, for example, be (i) a predetermined response that is greater than the sample response; (ii) a predetermined response that is less than the sample response; or (iii) a predetermined response that is independent of the concentration of analyte in the fluid sample.

To achieve a predetermined response that is greater than the control response, the second reagent composition can be a combination of the first reagent composition (or components thereof) and a supplemental reagent component that serves to increase (i.e., add to) the response of the control zone in comparison to the response of the sample detection zone. Such an "additive" supplemental reagent component can be, for example, the analyte. For example, if the analyte of interest in the fluid sample is glucose, the second reagent composition can be a combination that includes components of the first reagent composition and glucose in an amount that creates a desired predetermined control response that is greater than the sample response. Example 1 below includes an example of a second reagent composition that includes an "additive" supplemental reagent component.

On the other hand, to achieve a predetermined response that is less than the control response, the second reagent composition can, for example, be a combination of the first reagent composition (or components thereof) and a supplemental reagent component that serves to reduce (i.e., subtract from) the response of the control zone with respect to the sample response of the sample detection zone. Such "subtractive" supplemental reagent components can be, for example, reagent components that interact with (i) the analyte, (ii) components of the second reagent composition or (iii) intermediates (such as hydrogen peroxide) in a reaction sequence that produces the predetermined control response to prevent or lessen the response of the control zone. For the circumstance that the analyte is glucose and hydrogen peroxide linked oxidase colorimetric reagent compositions are employed, ascorbic acid or other reducing chemical species can be employed as a "subtractive" supplemental reagent component.

Since the second reagent composition of the control zone can, for example, be a combination of the first reagent composition of the sample detection zone and a supplemental reagent component, any reagent components that are common to both the sample and control zones can be present throughout matrix 104.

Finally, to obtain a predetermined control response that is independent of analyte concentration in the fluid sample, the second reagent composition can, for example, contain each of the components of the first reagent composition, including a dye(s), as well as the analyte. However, the dye(s) in the second reagent composition is present in a response limiting amount such that when the second reagent composition is exposed to the second portion of the fluid sample, the analyte present in the second reagent composition is sufficient to react with essentially all of the dye(s) in the second reagent composition to create the predetermined control response. Since the creation of the predetermined control response is, therefore, essentially a result of dye(s) and analyte present in the second reagent composition, the predetermined control response is independent of analyte in the fluid sample. Instead, the predetermined control response is dependent on the amount of dye(s) present in the second reagent composition. Such a second reagent composition is referred to as a "dye-limited" reagent composition since the amount of dye(s) determines the predetermined control response in the presence of the excess of analyte. Example 2 below provides an example of such a dye-limited second reagent composition.

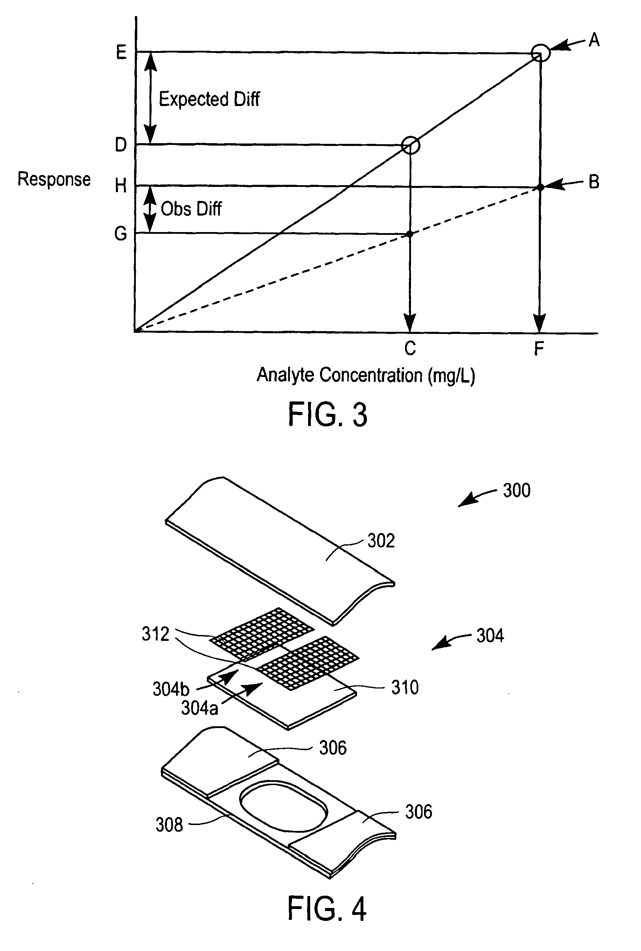

FIG. 3 is an idealized graph of analyte concentration (mg/L) in a fluid sample on the x-axis versus response (either a sample response or a predetermined control response) on the y-axis for a representative analytical strip according to the present invention wherein the second reagent composition is a combination of the first reagent composition and an "additive" supplemental reagent component (e.g., the analyte being determined). The solid line (line A) represents the expected relationship between analyte concentration and response (either sample response or predetermined control response) given that there are no interfering factors (such as, for example, degraded reagent components within the first and/or second reagent compositions, or matrix effects of the fluid sample). The dashed line (line B) represents a theoretical observed relationship between analyte concentration and response for the circumstance that an interfering factor(s) is present that decreases the sample and predetermined control responses.

Assuming that the analyte concentration in a fluid sample is "C" the expected sample response is "D." It is further assumed that the predetermined control response for the same fluid sample is "E" (corresponding to an analyte concentration of "F"). Such a predetermined control response could be created by, for example, including analyte in the second reagent composition equivalent to the difference between "F" and "C". However, in FIG. 3 the observed sample response is "G" and the observed predetermined control response is "H". The expected difference between the sample response and predetermined control response is the difference between "D" and "E" (i.e., the vertical arrow labeled "expected diff" in FIG. 3), while the observed difference is the difference between "G" and "H" (i.e., the vertical arrow labeled "obs diff" in FIG. 3).

For the circumstances of FIG. 3, a comparison of the observed difference and the expected difference can be employed to determine whether or not the analytical test strip and/or associated meter that detected those responses is functioning properly. In such a comparison, the expected difference would be known *a priori* based on the first and second reagent compositions. If, for example, the observed difference is equal to the expected difference within a predetermined tolerance, it can be deemed that the analytical test strip and associated meter are functioning properly. However, if the observed difference is not equal to the expected difference within the predetermined tolerance, it can be deemed that the analytical test strip and/or associated meter is not functioning reliably. In this manner, the control zone serves as an on-strip (i.e., "on-board") indicator of the reliability of a determination made using the analytical test strip.

Alternatively, the expected and observed differences can be used to adjust the sample response to account for interferences by use of, for example, the following algorithm:

$$ASP = SR \left(1 + ((ED - OD)/ED)\right)$$

where:

ASP is the adjusted sample response;
SR is the sample response;
ED is the expected difference; and
OD is the observed difference

In the algorithm, the factor $(1 + ((ED - OD)/ED))$ essentially serves as a calibrating factor for the analytical test strip.

Once apprised of the present disclosure, one skilled in the art will recognize that the use of a second reagent composition that creates a predetermined control response that is less than the sample response will also result in an expected difference and an observed difference that can be used to evaluate whether or not an analytical test strip and/or associated meter is functioning properly. For the circumstance where the second reagent composition creates a predetermined control response that is independent of analyte in the liquid sample, an observed control response can be compared to an expected predetermined control response as a measure of whether or not an analytical test strip and/or associated meter are functioning properly.

The measurement of sample and predetermined control responses, the calculation of observed response difference and the comparison of the observed response difference to an expected response difference can be accomplished using any suitable device(s) known to one skilled in the art. For example, such measurements and comparisons can be accomplished using hand-held meters and microprocessors and/or logic circuitry known to those skilled in the art.

Typical analytical test strips and their associated meters have a given dynamic range (i.e., the range over which an increase in analyte concentration gives a proportional increase in sample response). Therefore, it is conceivable that the use of a second reagent composition with an "additive" supplemental reagent component will result in a predetermined control response that is above the upper limit of the dynamic range when the liquid sample has a relatively high analyte concentration. It is also conceivable that the use of a second reagent composition with a "subtractive" supplemental reagent component will result in a predetermined control response that is below the lower limit of the dynamic range (typically zero) when the liquid sample has a relatively low analyte concentrations. Maximizing the additive supplemental reagent component or subtractive supplemental reagent component can be desirable since doing so can also maximize the signal-to-noise (S/N) ratio during comparison of sample and predetermined control responses. However, maximizing the additive or subtractive supplemental reagent component will also increase the likelihood of obtaining a predetermined control response that is outside of the dynamic range.

To remedy such a dynamic range issue, embodiments of analytical test strips according to the present invention can include a matrix with a sample detection zone, a first control zone and a second control zone. The sample detection zone includes a first reagent composition that reacts with analyte in the liquid sample to create a sample response and is configured to receive a first portion of the liquid sample. The first control zone includes a second reagent composition and is configured to receive a first fraction of a second portion of the liquid sample, while the second control zone includes a third reagent composition and is configured to receive a second fraction of the second portion of the liquid sample.

In such an embodiment, the second reagent composition reacts with the first fraction to create a first predetermined control response, while the third reagent composition reacts with the second fraction to create a second predetermined control response. Furthermore, the first predetermined control response is different from the second predetermined control response.

If the second reagent composition includes an "additive" supplemental reagent component and the third reagent composition includes a "subtractive" supplemental reagent component, the first and second predetermined control responses will differ from one another. In addition, at least one of the first and second predetermined control responses can be within the dynamic range regardless of whether the analyte concentration in the fluid sample is relatively high or relatively low.

FIG. 4 is a simplified exploded perspective view of an analytical test strip 300 for the determination of an analyte (i.e., glucose) in a liquid sample (i.e., whole blood) according to another exemplary embodiment of the present invention. Analytical test strip 300 includes a liquid sample spreading layer 302, a matrix 304, pressure sensitive adhesive layer 306 and base layer 308. In the depiction of FIG. 4, pressure sensitive adhesive layer 306 is shown adhered to base layer 308.

Matrix 304 of analytical test strip 300 includes both a membrane layer 310 and a screen layer 312. Furthermore, matrix 304 includes a sample detection zone 304a with a first reagent composition that reacts with analyte in the liquid sample to create a detectable sample response (for example, a colorimetric response) that is dependent on the concentration of analyte in the liquid sample. Sample detection zone 304a is configured to receive a first portion of a liquid sample that has been applied on liquid sample spreading layer 302 and includes both a portion of membrane layer 310 and a portion of screen layer 312.

Matrix 304 also includes a control zone 304b with a second reagent composition that creates a detectable predetermined control response (for example, a predetermined colorimetric response) when exposed to a second portion of the liquid sample. Control zone 304b is configured to receive a second portion of the liquid sample that has been applied on liquid sample spreading layer 302.

It should be noted that components of the first and second reagent compositions can be present prior to use of analytical tests strip 300 either on membrane layer 310 or on screen layer 312. As a liquid sample is transferred from liquid sample spreading layer 302 to membrane layer 310 across screen layer 312, first and second reagent components present in screen layer 312 are dissolved in the liquid sample and transferred to membrane layer 310.

Liquid sample spreading layer 302 serves to spread a liquid sample across matrix 304 such that a first portion of the liquid sample is transferred to sample detection zone 304a, while a second portion of the liquid sample is transferred to control zone 304b.

**EXAMPLES**

**Example 1 — Analytical Test Strip with a control zone a second reagent composition with "additive" supplemental reagent component.**

Analytical test strips for the determination of glucose in a whole blood sample were prepared using the following solutions:

Solution A (also referred to as an "enzymes, buffers, and stabilizers solution")

> 10 ml water
> 112.8 mg citric acid, monohydrate
> 139.2 mg sodium citrate, dehydrate
> 100 mg mannitol
> 8.4 mg disodium EDTA
> 45 mg Gantrez S95
> 168.3 mg Crotien SPA
> 1100 IU glucose oxidase
> 617 IU horseradish peroxidase
> 0.5 ml (11% w/v Carbopol 910 suspended in acetonitrile)
> 1.5 ml (0.1M citrate, pH 5.0)

Solution B1 (also referred to as a "dye solution")

> 10 mL (52.5:17.5:30 EtOH:MeOH:$H_2O$)
> 40.9 mg N-[sulfonyl-m-sodium benzenesulfonate]-3-methyl-2-benzothiazolinone hydrazone (MBTH-SBS)
> 56.6 mg 8-anilino-1-naphthalenesulfonic acid, ammonium salt (ANS)
> 0.48 mL (20% w/v Maphos 60A in 52.5:17.5:30 EtOH:MeOH:$H_2O$)

Solution C1 (also referred to as a "dye and glucose solution")

> 10 ml MeOH
> 36.8 mg MBTH-SBS
> 60.8 mg ANS
> 32 mg β-d-glucose

To prepare the analytical test strips, Solution A was coated on a matrix (namely, an asymmetrical BTS30 polysulfone membrane available commercially from US Filter) by passing the matrix, large pore side down, over a trough containing Solution A such that the matrix wicked-up Solution A. Excess Solution A was then removed from the matrix by passing the matrix over a scraping bar. The matrix was then dried in a forced air dryer for approximately 3 minutes at 79 °C.

Solution B1 was subsequently coated on the matrix and dried in the same manner as was done for Solution A. Thereafter, the matrix was slit into 0.635 cm (¼ inch) sections to provide a matrix impregnated with Solution A and Solution B1. It should be noted that the combination of Solution A and Solution B1 serves as a first reagent composition that creates a colorimetric sample response upon reaction with glucose in a blood sample. One skilled in the art will recognize the combination of Solution A and Solution B1 as exemplary of a hydrogen peroxide linked oxidase colorimetric reagent composition.

The ¼ inch sections were striped with solution C by a slot die process using a coating head with channels that directed fluid to 0.127 mm (0.005 inch) wide orifices spaced at 1.524 mm (0.060 inch) perpendicular to the length of a matrix. To prepare the analytical test strips of this example, only one orifice was used. With the orifices facing upward, the matrix, with the large pore side up, was pulled over the coating head at 2.79 cm/sec (5.5 ft/ minute). Solution C1 was fed into the coating head by means of a syringe pump operating at 0.8 μL/minute while the matrix was dried by a heat gun. After drying, the matrix was creamy white, with no other visible color.

It should be noted that the combination of Solutions A, B1 and C1 serves as a second reagent composition that creates a colorimetric predetermined control response upon exposure to a blood sample. Since reaction between glucose in Solution C1 and components of Solutions A and B1 must be prevented prior to application of a blood sample to the analytical test strip, methanol (which does not activate the enzymes present in dried Solution A) was employed in Solution C1 rather than water.

After drying, 0.635 cm (¼ inch) by 0.635 cm (¼ inch) pieces of the matrix were affixed to 0.635 cm (¼ inch) wide pieces of 0.036 cm (0.014 inch) thick Melinex 329 film that functioned as a base layer and that had an opening. A 2.54 cm (1 inch) by 0.635 cm (¼ inch) piece of porous material (i.e., a porous polyethylene material available from Porex, Fairburn GA) was then placed on top of the membrane to serve as a liquid sample spreading layer and adhered to the base layer with a double-sided adhesive layer. The resulting analytical test strip had a stripe-shaped control zone that was perpendicular to a longitudinal axis of the analytical test strip.

Analytical test strips prepared as noted above were tested using aliquots of whole blood (at a hematocrit of 42%) that had been adjusted to 51, 81 and 191 mg/dl of glucose by adding concentrated aqueous d-glucose. The testing was conducted by placing whole blood samples directly onto the liquid sample spreading layer above the matrix. The whole blood samples transferred into the matrix and excess whole blood sample was wicked into the liquid sample spreading layer.

After a 45 second development, the analytical test strips were inserted into a measuring device. The measuring device included a reflectometer based on a commercially available Agilent HEDS 1500 barcode detector. The measuring device included circuitry that (i) operated an LED/photodetector couple in the barcode detector and (ii) communicated the detector output to a personal computer via an A/D converter. The measuring device also included a fixture that aligned the analytical test strips at a 15 degree angle to the barcode detector, with the plane of the matrix of the analytical test strips coinciding with a focal point of the barcode detector.

As each analytical test strip was inserted into the fixture, the barcode detector scanned the analytical test strip across the bottom (namely, small pore) side of the matrix. In doing so, the barcode detector scanned across to the matrix (which was exposed through an opening in the base layer) and thus across the sample and control zones of the matrix. The detector output was converted to relative reflectance (R) by calculating a ratio of the detector output to a detector output obtained from the base layer of the analytical test strips. Relative reflectance (R) was then converted to K/S (a quantity known in the art to be proportional to light absorbing components of a scattering medium) according to the following relationship:

$$K/S = \frac{(1-R)^2}{2R}$$

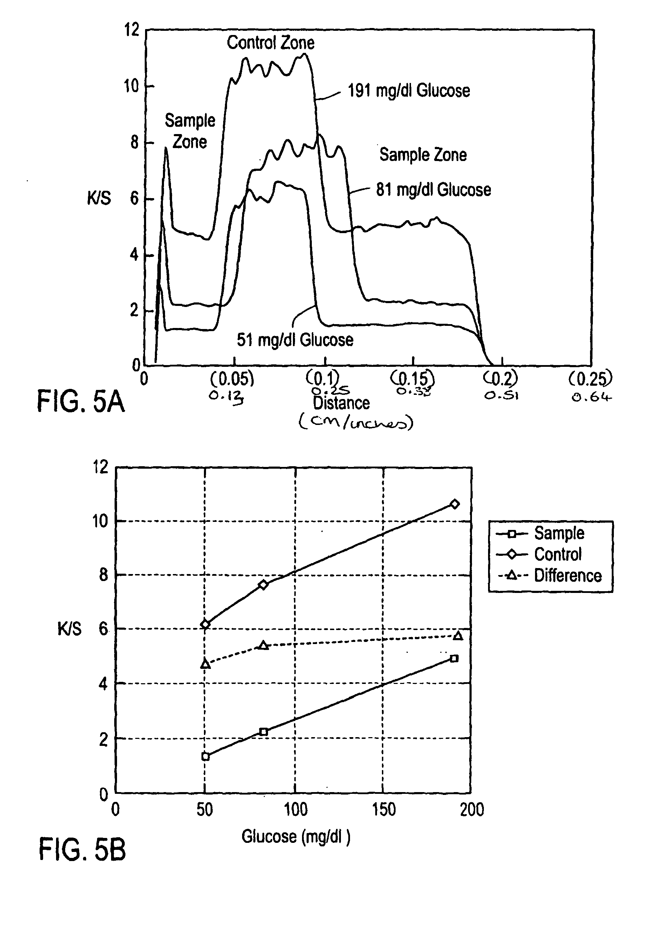

The measuring device recorded an optical scan of the analytical test strips as the analytical test strips passed over the measuring device's detector. The scanned data were then converted to K/S as described above. FIG. 5A depicts scans of individual analytical test strips at the three glucose levels of 51, 81 and 191 mg/dl. In FIG. 5A, the response on either side of the central peak are sample responses created in the sample detection zones of the analytical test strip (i.e., the sample detection zones on either side of the stripe-shaped control zone). In FIG. 5A, the control zone response is between the sample detection zone responses and is created by the exposure of the second reagent composition (i.e., a combination of Solutions A, B1 and C1) to a portion of the whole blood sample.

FIG. 5B (a plot of K/S versus glucose concentration for the sample detection zone, control zone and difference therebetween) demonstrates that the difference between the predetermined control response (as represented by K/S) and sample response (also as represented by K/S) is essentially constant at each of the tested glucose levels.

**Example 2 - Analytical test strip with control zone that creates a predetermined control response when exposed to a fluid sample that is independent of analyte concentration in the fluid sample.**

Analytical test strips for the determination of glucose in a whole blood sample were prepared using the following solutions:

Solution A (also referred to as an "enzymes, buffers, and stabilizers solution")
The composition of Solution A in example 2 was identical to Solution A in Example 1

Solution B2 (also referred to as "dye solution B2")

10 ml (52.5:17.5:30 EtOH:MeOH:H$_2$O)
174.6 mg MBTH-SBS
271 mg ANS

Solution C2 (also referred to as "dye and glucose solution C2")

  10 ml EtOH
  300 mg β-d-glucose
  23.3 mg MBTH-SBS
  39.7 mg ANS

To prepare the analytical test strips of this example, Solution A was coated onto a BTS-30 membrane (i.e., the matrix) in the same manner as Solution A was applied in Example 1 above. Solutions B2 and C2 were striped onto the large pore side of the matrix in the same manner as solution C1 in Example 1, except that Solutions B2 and C2 were striped on simultaneously through orifices spaced 0.154 cm (0.060 inch) apart. The striping was done at a speed of 2.79 cm/sec (5.5 ft/min), a flow rate of 1.0 μL/sec. and a temperature of 95 °C. The analytical test strips were then further prepared in the same fashion as Example 1. This manner of preparation resulted in a matrix that included a sample detection zone that included a first reagent prepared from Solutions A and B2 and a control zone that included a second reagent composition prepared from Solutions A, B2 and C2.

It should be noted that the combination of Solution A and Solution B2 serves as a first reagent composition that creates a colorimetric sample response upon reaction with glucose in a blood sample. One skilled in the art will recognize the combination of Solution A and Solution B2 as exemplary of a hydrogen peroxide linked oxidase colorimetric reagent composition.

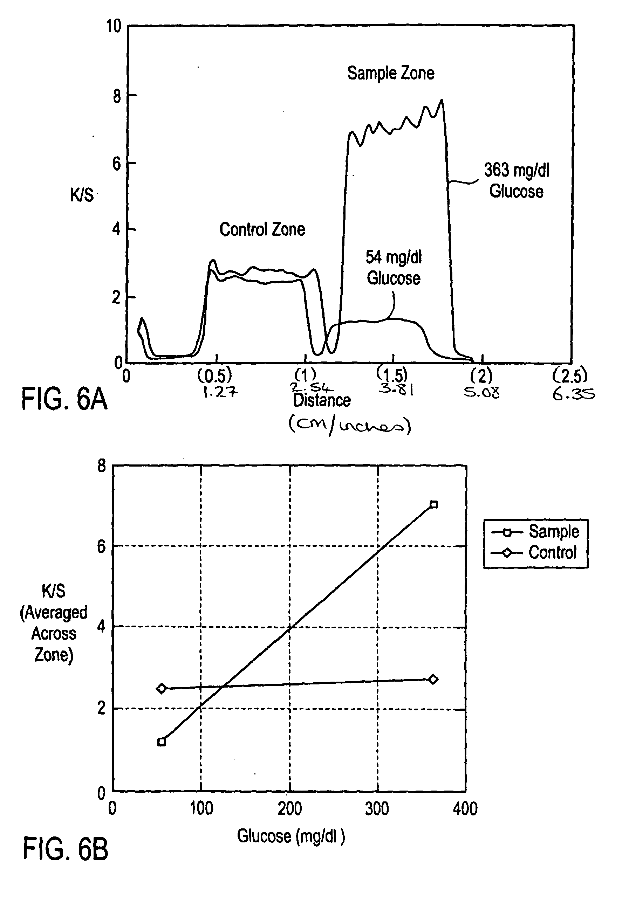

Analytical test strips prepared as described where then tested with aliquots of whole blood (with a hematocrit level of 42%) that had been adjusted to glucose concentrations of 54 mg/dl and 363 mg/dl. The testing was otherwise conducted as described above with respect to Example 1. FIGs. 6A and 6B depict the results of scans across the control zone and sample detection zone of analytical test strips subjected to the whole blood aliquots. Although the response of the sample detection zone is dependent on glucose concentration in the whole blood aliquots, the predetermined control response of the control zone is essentially constant and independent of the glucose concentration in the whole blood aliquots.

Once apprised of the present disclosure, one skilled in the art will recognize that analytical tests strips according to the present invention can be, for example, electrochemical-based analytical test strips. In this circumstance, the sample response and predetermined control responses would be electrochemical responses.

It is envisioned that the predetermined control response and sample response obtained from analytical test strips according to the present invention could be employed to determine whether or not the sample zone and/or control zone have been adequately filled with liquid sample. Such a determination could be made by, for example, comparing an observed difference between the predetermined control and sample responses to an expected difference.

In addition, the inclusion of (i) a blank zone (i.e., a zone that exhibits a "blank" response equivalent to zero analyte concentration in the liquid sample) in combination with (ii) a control zone that employs an additive supplemental reagent component in the second reagent composition can enable a response slope and response intercept to be determined. Such a determination would be based on the blank response and predetermined control response. The response slope and response intercept could then be used to obtain calibration factor(s) for the analytical test strip and/or to verify that a correct calibration code is being employed by an associated device (e.g., a meter).

Embodiments of analytical test strips according to the present invention can be configured for the analysis of multiple analytes in a liquid sample by employing a plurality of sample zones and a plurality of associated control zones. The reagent composition in each of the sample zones would be adapted to create a response for a specific analyte (e.g., glucose or ketones) and the reagent composition of the associated control zone would be adapted to create predetermined control responses when exposed to the liquid sample. In this circumstance, manufacturing of the analytical test strips can be simplified if any reagent components common to the plurality of sample zones and plurality of associated sample zones are present throughout the analytical test strip's matrix.

Embodiments of analytical test strips according to the present invention can also include a reference zone that is not exposed to the liquid sample. Such a reference zone can be used, for example, to provide a standard reflectance response even after a liquid sample has been applied to the analytical test strip. Furthermore, a white-colored zone that receives a portion of the liquid sample can be provided and adapted such that a response of the white-colored zone is useful in evaluating characteristics of the liquid sample (e.g., evaluating the hematocrit of a blood sample).

Embodiments of analytical test strips according to the present invention can be configured such that the sample zone and control zone can be scanned for their respective responses in a linear fashion and the sample and control zone detected by a suitable signal processing technique (e.g., peak detection signal processing techniques). Such linear scanning can also reduce a required registration between the analytical test strips and an associated meter, with the reduced registration being beneficial in terms of minimizing the volume of liquid sample required to successfully employ the analytical test strip.

It should be understood that various alternatives to the embodiments of the invention described herein may be employed in practicing the invention. It is intended that the following claims define the scope of the invention and that structures within the scope of these claims and their equivalents be covered thereby.

**Claims**

1. An analytical test strip for the determination of an analyte in a liquid sample, the analytical test strip comprising:

   a matrix comprising a membrane and a screen layer, the matrix including:

   a sample detection zone with a first reagent composition that reacts with analyte in the liquid sample to create a sample response, the sample detection zone configured to receive a first portion of the liquid sample; and at least one control zone with a second reagent composition, the at least one control zone configured to receive a second portion of the liquid sample,

   wherein the second reagent composition creates a predetermined control response when exposed to the second portion of the liquid sample, and

   wherein components of the second reagent composition are coated on the membrane and on the screen layer.

2. The analytical test strip of claim 1, wherein the first reagent composition and the second reagent composition are coated directly on the matrix membrane.

3. The analytical test strip of claim 1, wherein the sample response and predetermined control response are colorimetric responses.

4. The analytical test strip of claim 1, wherein the predetermined control response is greater than the sample response.

5. The analytical test strip of claim 4, wherein the second reagent composition is a combination that includes components of the first reagent composition and the analyte.

6. The analytical test strip of claim 5, wherein the liquid sample is whole blood and the analyte is glucose.

7. The analytical test strip of claim 6, wherein the predetermined control response is less than the sample response.

8. The analytical test strip of claim 1, wherein the predetermined control response is independent of analyte concentration in the fluid sample.

9. The analytical test strip of claim 8, wherein the second reagent composition includes at least one dye and the predetermined control response is a dye-limited response.

10. The analytical test strip of claim 1, wherein the second reagent composition includes an additive supplemental reagent component wherein the additive supplemental reagent component increases the response of the control zone in comparison to the response of the sample detection zone.

11. The analytical test strip of claim 10, wherein the analyte is glucose and the additive supplemental reagent component is glucose, wherein the additive supplemental reagent component increases the response of the control zone in comparison to the response of the sample detection zone.

12. The analytical test strip of claim 1, wherein the second reagent composition includes a subtractive supplemental reagent component, wherein the subtractive supplemental reagent component decreases the response of the control zone in comparison to the response of the sample detection zone.

13. The analytical test strip of claim 12, wherein the analyte is glucose and the subtractive supplemental reagent component is ascorbic acid, wherein the subtractive supplemental reagent component decreases the response of the control zone in comparison to the response of the sample detection zone.

14. The analytical test strip of claim 13, wherein the first reagent composition and the second reagent composition are hydrogen peroxide linked oxidase colorimetric reagent compositions.

15. An analytical test strip for the determination of an analyte in a liquid sample, the analytical test strip comprising:

   a matrix comprising a membrane and a screen layer, the matrix including:

   a sample detection zone with a first reagent composition that reacts with analyte in the liquid sample to create a sample response, the sample detection zone configured to receive a first portion of the liquid sample; a first control zone with a second reagent composition and configured to receive a first fraction of a second portion of the liquid sample; and a second control zone with a third reagent composition and configured to receive a second fraction of the second portion of the liquid sample,

wherein the second reagent composition reacts with the first fraction of the second portion of the liquid sample to create a first predetermined control response,

wherein the third reagent composition reacts with the second fraction of the second portion of the liquid sample to create a second predetermined control response, and

wherein the first predetermined control response is different than the second predetermined control response, and

wherein components of the second reagent composition and the third reagent composition are coated on the membrane and on the screen layer.

16. The analytical test strip of claim 15, wherein the second reagent composition includes an additive supplemental reagent component and the third reagent composition includes a subtractive supplemental reagent component,

wherein the subtractive supplemental reagent component decreases the response of the control zone in comparison to the response of the sample detection zone, and

wherein the additive supplemental reagent component increases the response of the control zone in comparison to the response of the sample detection zone.

17. The analytical test strip of claim 16, wherein the analyte is glucose, the additive supplemental reagent component is glucose and the subtractive supplemental reagent component is ascorbic acid,

wherein the subtractive supplemental reagent component decreases the response of the control zone in comparison to the response of the sample detection zone, and

wherein the additive supplemental reagent component increases the response of the control zone in comparison to the response of the sample detection zone.

18. The analytical test strip of claim 17, wherein the first reagent composition and the second reagent compositions are hydrogen peroxide linked oxidase colorimetric reagent compositions.

**Patentansprüche**

1. Analytischer Teststreifen zum Bestimmen eines Analyts in einer flüssigen Probe, wobei der analytische Teststreifen umfasst:

eine Matrix, die eine Membran und eine Screening-Schicht aufweist, wobei die Matrix enthält:

eine Probendetektionszone mit einer ersten Reagenz-Zusammensetzung, die mit Analyten in der flüssigen Probe reagiert, um eine Probenantwort zu erzeugen, wobei die Probendetektionszone gestaltet ist, um einen ersten Teil der flüssigen Probe aufzunehmen; und

wenigstens eine Kontrollzone mit einer zweiten Reagenz-Zusammensetzung, wobei die wenigstens eine Kontrollzone gestaltet ist, um einen zweiten Teil der flüssigen Probe aufzunehmen,

wobei die zweite Reagenz-Zusammensetzung eine vorbestimmte Kontrollantwort erzeugt, wenn sie dem zweiten Teil der flüssigen Probe ausgesetzt wird, und

wobei Komponenten der zweiten Reagenz-Zusammensetzung auf der Membran und auf die Screening-Schicht aufgebracht sind.

2. Analytischer Teststreifen nach Anspruch 1, **dadurch gekennzeichnet, dass** die erste Reagenz-Zusammensetzung und die zweite Reagenz-Zusammensetzung direkt auf die Matrixmembran ausgebracht sind.

3. Analytischer Teststreifen nach Anspruch 1, **dadurch gekennzeichnet, dass** die Probenantwort und vorbestimmte Kontrollantwort kolorimetrische Antworten sind.

4. Analytischer Teststreifen nach Anspruch 1, **dadurch gekennzeichnet, dass** die vorbestimmte Kontrollantwort größer als die Probenantwort ist.

5. Analytischer Teststreifen nach Anspruch 4, **dadurch gekennzeichnet, dass** die zweite Reagenz-Zusammensetzung eine Kombination ist, die Komponenten der ersten Reagenz-Zusammensetzung und des Analyts aufweist.

6. Analytischer Teststreifen nach Anspruch 5, **dadurch gekennzeichnet, dass** die flüssige Probe Vollblut und das Analyt Glukose ist.

7. Analytischer Teststreifen nach Anspruch 6, **dadurch gekennzeichnet, dass** die vorbestimmte Kontrollantwort geringer als die Probenantwort ist.

8. Analytischer Teststreifen nach Anspruch 1, **dadurch gekennzeichnet, dass** die vorbestimmte Kontrollantwort unabhängig von Analytenkonzentration in der flüssigen Probe ist.

9. Analytischer Teststreifen nach Anspruch 8, **dadurch gekennzeichnet, dass** die zweite Reagenz-Zusammensetzung wenigstens einen Farbstoff aufweist und die vorbestimmte Kontrollantwort eine Farbstoff-beschränkte Antwort ist.

10. Analytischer Teststreifen nach Anspruch 1, **dadurch gekennzeichnet, dass** die zweite Reagenz-Zusammensetzung eine additive ergänzende Reagenz-Komponente aufweist, wobei die additive ergänzende Reagenz-Komponente die Antwort der Kontrollzone im Vergleich zu der Antwort der Probendetektionszone steigert.

11. Analytischer Teststreifen nach Anspruch 10, **dadurch gekennzeichnet, dass** das Analyt Glukose ist und die additive ergänzende Reagenz-Komponente Glukose ist,
wobei die additive ergänzende Reagenz-Komponente die Antwort der Kontrollzone im Vergleich zu der Antwort der Probendetektionszone steigert.

12. Analytischer Teststreifen nach Anspruch 1, **dadurch gekennzeichnet, dass** die zweite Reagenz-Zusammensetzung eine subtraktive ergänzende Reagenz-Komponente aufweist,
wobei die subtraktive ergänzende Reagenz-Komponente die Antwort der Kontrollzone im Vergleich zu der Antwort der Probendetektionszone verringert.

13. Analytischer Teststreifen nach Anspruch 12, **dadurch gekennzeichnet, dass** das Analyt Glukose ist und die subtraktive ergänzende Reagenz-Komponente Ascorbinsäure ist,
wobei die subtraktive ergänzende Reagenz-Komponente die Antwort der Kontrollzone im Vergleich mit der Antwort der Probendetektionszone verringert.

14. Analytischer Teststreifen nach Anspruch 13, **dadurch gekennzeichnet, dass** die erste Reagenz-Zusammensetzung und die zweite Reagenz-Zusammensetzung kolorimetrische Reagenz-Zusammensetzungen mit Wasserstoffperoxid-verbundener Oxidase sind.

15. Analytischer Teststreifen zur Bestimmung eines Analyts in einer flüssigen Probe, wobei der analytische Teststreifen umfasst:

eine Matrix, die eine Membran und eine Screening-Schicht aufweist, wobei die Matrix aufweist:

eine Probendetektionszone mit einer ersten Reagenz-Zusammensetzung, die mit Analyten in der flüssigen Probe reagiert, um eine Probenantwort zu erzeugen, wobei die Probendetektionszone gestaltet ist, um einen ersten Teil der flüssigen Probe aufzunehmen;
eine erste Kontrollzone mit einer zweiten Reagenz-Zusammensetzung, die gestaltet ist, um einen ersten Anteil eines zweiten Teils der flüssigen Probe aufzunehmen; und
eine zweite Kontrollzone mit einer dritten Reagenz-Zusammensetzung, die gestaltet ist, um einen zweiten Anteil des zweiten Teils der flüssigen Probe aufzunehmen;

wobei die zweite Reagenz-Zusammensetzung mit dem ersten Anteil des zweiten Teils der flüssigen Probe reagiert, um eine erste eine vorbestimmte Kontrollantwort zu erzeugen,
wobei die dritte Reagenz-Zusammensetzung mit dem zweiten Anteil des zweiten Teils der flüssigen Probe reagiert, um eine zweite vorbestimme Kontrollantwort zu erzeugen, und
wobei sich die erste vorbestimmte Kontrollantwort von der zweiten vorbestimmten Kontrollantwort unterscheidet, und
wobei Komponenten der zweiten Reagenz-Zusammensetzung und der dritten Reagenz-Zusammensetzung auf der Membran und auf die Auswertungsschicht aufgebracht sind.

16. Analytischer Teststreifen nach Anspruch 15, **dadurch gekennzeichnet, dass** die zweite Reagenz-Zusammensetzung eine additive ergänzende Reagenz-Komponente aufweist und die dritte Reagenz-Zusammensetzung eine subtraktive ergänzende Reagenz-Komponente aufweist,
wobei die subtraktive ergänzende Reagenz-Komponente die Antwort der Kontrollzone im Vergleich zu der Antwort der Probendetektionszone verringert, und
wobei die additive ergänzende Reagenz-Komponente die Antwort der Kontrollzone im Vergleich zu der Antwort der Probendetektionszone steigert.

17. Analytischer Teststreifen nach Anspruch 16, **dadurch gekennzeichnet, dass** das Analyt Glukose ist, die additive ergänzende Reagenz-Komponente Glukose ist und die subtraktive ergänzende Reagenz-Komponente Ascorbinsäure ist,
wobei die subtraktive ergänzende Reagenz-Komponente die Antwort der Kontrollzone im Vergleich zu der Antwort der Probendetektionszone verringert, und
wobei die additive ergänzende Reagenzkomponente die Antwort der Kontrollzone im Vergleich zu der Antwort der Probendetektionszone steigert.

18. Analytischer Teststreifen nach Anspruch 17, **dadurch gekennzeichnet, dass** die erste Reagenz-Zusammensetzung und die zweiten Reagenz-Zusammensetzungen kolorimetrische Reagenz-Zusammensetzungen mit Wasserstoffperoxid-verbundener Oxidase sind.

**Revendications**

1. Bande de test analytique, pour la détermination d'un analyte dans un échantillon liquide, la bande de test analytique comprenant :

    ■ une matrice comprenant une membrane et une couche écran, la matrice comprenant

        ■ une zone de détection d'échantillon présentant une première composition de réactif qui réagit avec un analyte dans l'échantillon liquide pour créer une réponse d'échantillon, la zone de détection de l'échantillon étant configurée pour recevoir une première partie de l'échantillon liquide ; et
        ■ au moins une zone de contrôle présentant une deuxième composition de réactif, l'au moins une zone de contrôle étant configurée pour recevoir une deuxième partie de l'échantillon liquide,

            ■ la deuxième composition de réactif créant une réponse témoin prédéterminée lorsqu'elle est exposée à la deuxième partie de l'échantillon liquide, et
            ■ les composants de la deuxième composition de réactif étant appliqués sur la membrane et sur la couche écran.

2. Bande de test analytique selon la revendication 1, dans laquelle la première composition de réactif et la deuxième composition de réactif sont appliquées directement sur la membrane de matrice.

3. Bande de test analytique selon la revendication 1, dans laquelle la réponse de l'échantillon et la réponse témoin prédéterminée sont des réponses colorimétriques.

4. Bande de test analytique selon la revendication 1, dans laquelle la réponse témoin prédéterminée est supérieure à la réponse de l'échantillon.

5. Bande de test analytique selon la revendication 4, dans laquelle la deuxième composition de réactif est une combinaison qui comprend des composants de la première composition de réactif et l'analyte.

6. Bande de test analytique selon la revendication 5, dans laquelle l'échantillon de liquide est du sang complet et l'analyte est du glucose.

7. Bande de test analytique selon la revendication 6, dans laquelle la réponse témoin prédéterminée est inférieure à la réponse de l'échantillon.

8. Bande de test analytique selon la revendication 1, dans laquelle la réponse témoin prédéterminée est indépendante de la concentration d'analyte dans l'échantillon fluide.

9. Bande de test analytique selon la revendication 8, dans laquelle la deuxième composition de réactif comprend au moins un colorant et la réponse témoin prédéterminée est une réponse limitée au colorant.

10. Bande de test analytique selon la revendication 1, dans laquelle la deuxième composition de réactif comprend un composant de réactif additif supplémentaire, le composant de réactif additif supplémentaire augmentant la réponse de la zone de contrôle par rapport à la réponse de la zone de détection d'échantillon.

11. Bande de test analytique selon la revendication 10, dans laquelle l'analyte est du glucose et le composant de réactif additif supplémentaire est du glucose, le composant de réactif additif supplémentaire augmentant la réponse de la zone de contrôle par rapport à la réponse de la zone de détection de l'échantillon.

12. Bande de test analytique selon la revendication 1, dans laquelle la deuxième composition de réactif comprend un composant de réactif soustractif supplémentaire, le composant de réactif soustractif supplémentaire diminuant la réponse de la zone de contrôle par rapport à la réponse de la zone de détection de l'échantillon.

13. Bande de test analytique selon la revendication 12, dans laquelle l'analyte est du glucose et le composant de réactif soustractif supplémentaire est de l'acide ascorbique, le composant de réactif soustractif supplémentaire diminuant la réponse de la zone de contrôle par rapport à la réponse de la zone de détection d'échantillon.

14. Bande de test analytique selon la revendication 13, dans laquelle la première composition de réactif et la deuxième composition de réactif sont des compositions réactives colorimétriques oxydase liées au peroxyde d'hydrogène.

15. Bande de test analytique pour la détermination d'un analyte dans un échantillon liquide, la bande de test analytique comprenant :

    ■ une matrice comprenant une membrane et une couche écran, la matrice comprenant

        ■ une zone de détection d'échantillon présentant une première composition de réactif qui réagit avec un analyte dans l'échantillon liquide pour créer une réponse d'échantillon, la zone de détection de l'échantillon étant configurée pour recevoir une première partie de l'échantillon liquide ;

■ une première zone de contrôle présentant une deuxième composition de réactif et configurée pour recevoir une première fraction d'une deuxième partie de l'échantillon liquide ; et

■ une deuxième zone de contrôle présentant une troisième composition de réactif et configurée pour recevoir une deuxième fraction de la deuxième partie de l'échantillon liquide,

■ la deuxième composition de réactif réagissant avec la première fraction de la deuxième partie de l'échantillon liquide pour créer une première réponse témoin prédéterminée,

■ la troisième composition de réactif réagissant avec la deuxième fraction de la deuxième partie de l'échantillon liquide pour créer une deuxième réponse témoin prédéterminée et

■ la première réponse témoin prédéterminée étant différente de la deuxième réponse témoin prédéterminée, et

■ les composants de la deuxième composition de réactif et la troisième composition de réactif étant appliqués sur la membrane et sur la couche écran.

16. Bande de test analytique selon la revendication 15, dans laquelle la deuxième composition de réactif comprend un composant de réactif additif supplémentaire et la troisième composition de réactif comprend un composant de réactif soustractif supplémentaire

■ le composant de réactif soustractif supplémentaire diminuant la réponse de la zone de contrôle par rapport à la réponse de la zone de détection d'échantillon, et

■ le composant de réactif additif supplémentaire augmentant la réponse de la zone de contrôle par rapport à la réponse de la zone de détection d'échantillon.

17. Bande de test analytique selon la revendication 16, dans laquelle l'analyte est du glucose et le composant de réactif soustractif supplémentaire est de l'acide ascorbique,

■ le composant de réactif soustractif supplémentaire diminuant la réponse de la zone de contrôle par rapport à la réponse de la zone de détection d'échantillon, et

■ le composant de réactif additif supplémentaire augmentant la réponse de la zone de contrôle par rapport à la réponse de la zone de détection d'échantillon.

18. Bande de test analytique selon la revendication 17, dans laquelle la première composition de réactif et les deuxièmes compositions de réactif sont des compositions de réactif colorimétrique oxydase liées au peroxyde d'hydrogène.

100

102
~~802~~

104b

104a

104

108b

106

108

106

## FIG. 1

200b   200a

200

104a

104b

104

Web Direction

## FIG. 2

**FIG. 3**

**FIG. 4**

FIG. 5A

FIG. 5B

FIG. 6A

FIG. 6B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5304468 A
- US 6162397 A
- US 6168957 B
- US 4900666 A
- US 5902731 A
- US 5968836 A
- US 4935346 A